(19) Europäisches Patentamt
European Patent Office
Office européen des brevets

(11) Numéro de publication: **0 158 556**
**B1**

(12) # FASCICULE DE BREVET EUROPEEN

(45) Date de publication du fascicule du brevet: **01.07.87**

(51) Int. Cl.$^4$: **C 07 C 99/00,** C 07 C 101/12

(21) Numéro de dépôt: **85400567.5**

(22) Date de dépôt: **22.03.85**

(54) **Procédé de préparation d'acides hydroxyalkylaminoacétiques.**

(30) Priorité: **26.03.84 FR 8404656**

(43) Date de publication de la demande:
**16.10.85 Bulletin 85/42**

(45) Mention de la délivrance du brevet:
**01.07.87 Bulletin 87/27**

(84) Etats contractants désignés:
**BE DE GB NL SE**

(58) Documents cités:

**BULL. SOC. CHIM. FR, janvier-février 1978, partie II, pages 83-88, Paris, FR; P.A. LAURENT et al.: "Etudes sur les acétals mixtes cycliques. XVIII. Réactions du glyoxal avec les amino-alcools"**

(73) Titulaire: **SOCIETE FRANCAISE HOECHST
Société anonyme dite:
3, avenue du Général de Gaulle
F-92800 Puteaux (FR)**

(72) Inventeur: **Blanc, Alain
21Bis rue Galvanis
F-75017 Paris (FR)**

(74) Mandataire: **Rinuy, Santarelli
14, avenue de la Grande Armée
F-75017 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 158 556 B1

Courier Press, Leamington Spa, England.

**Description**

La présente invention concerne un procédé de préparation d'acides hydroxyalkylamioacétiques.

Les acides hydroxyalkylaminoacétiques obtenus par le procédé de la présente invention sont les produits de formule générale (I):

$$—R—CHOH—CHR_1—NR_2—CH_2—COOH \qquad (I)$$

dans laquelle:

R représente un atome d'hydrogène, un groupement alkyle en $C_1—C_{18}$, a chaîne droite ou ramifée ou un groupement phényle éventuellement substitué par un ou plusieurs radicaux (de préférence un ou deux) choisis parmi les radicaux: alkyle en $C_1—C_4$, alcoxyle en $C_1—C_4$ ou halogéno;

$R_1$ représente un atome d'hydrogène ou un groupement alkyle en $C_1—C_4$;

$R_2$ représente un groupement alkyle en $C_1—C_{18}$ ou le groupement $R_3—CHOH—CHR_4—$ dans lequel $R_3$, identique à R, représente un atome d'hydrogène ou un groupement alkyle en $C_1—C_{18}$, à chaîne droite ou ramifiée et $R_4$ est identique à $R_1$.

Les groupes substituants décrits ci-dessus peuvent, en outre, être définis comme suit:

un radical halogéno comprend le chlore, le brome, le fluor et l'iode. Les substituants halogéno préférés sont le chlore et le fluor;

un groupement alkyle en $C_1—C_{18}$, à chaîne droite ou ramifiée, comprend, par exemple, les radicaux méthyle, éthyle, propyle, n-butyle, hexyle, nonyle.

De façon avantageuse, la présente invention couvre un procédé de préparation des produits de formule (II):

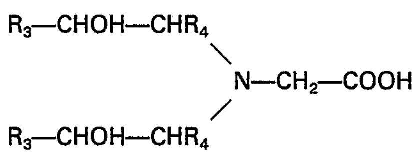

(II)

dans laquelle $R_3$ représente un atome d'hydrogène ou un radical alkyle en $C_1—C_{18}$ et $R_4$ représente un atome d'hydrogène ou un radical alkyle en $C_1—C_4$.

L'invention a tout spécialement pour objet un procédé de préparation des composés suivants:

—N,N-bis(hydroxy-2 éthyl)glycine;

—N,N-bis(hydroxy-2)propyl glycine;

—N-méthyl N-(hydroxy-2 éthyl)glycine;

—N-méthyl N-(hydroxy-2 méthyl-1 phényl-2 éthyl)glycine.

De nombreux produits de formule générale (I) sont décrits ou peuvent être obtenus par des moyens connus, et notamment la N,N-bis (hydroxy-2 éthyl)glycine qui est largement utilisée pour ses propriétés chélatantes ou complexantes.

On sait accéder à la N,N-bis(hydroxy-2 éthyl)glycine par:

—condensation de l'oxyde d'éthylène avec la glycine selon A. J. Kippianov, Ukrain Khim, Zhur., 1926, *2*, 236;

hydrolyse du N,N-bis(hydroxy-2 éthyl) acétonitrile obtenu par réaction de Strecker sur la diéthanolamine (V. F. Lyubomudrov, Ukrain Khim. Zhur. 1936, *11*, 119; A. E. Frost et al., J. Amer. Chem. Soc., 1957, *79*, 2755-58; brevets des Etats Unis d'Amérique N° 2.845.457 et 2.860.164);

condensation de l'acide chloroacétique avec la diéthanol-amine selon N. V. Khromov-Borisov et al. Zhur. obshchei Khim., 1953, *23*, 598 et W. S. Gump et al., J. Org. Chem., 1959, *24*, 712-14;

hydrolyse de la (hydroxy-2 éthyl)-4 morpholinone-2, produit secondaire mais parfois aussi majoritaire, obtenue lors de la mise en oeuvre des procédés précédents (M. L. Pascal, Bull. Soc. Chim. France, 1960, 435-42 et Compt. Rend., 1957, *245*, 1318-20; N. V. Khromov-Borisov et al. loc. cit.).

Ces méthodes sont générales et elles peuvent, sous certaines conditions, être étendues à la préparation des produits de formule générale (I) par un choix judicieux des matières premières. Tel est le cas, par exemple, de la cyanométhylation des amines secondaires hydroxylées selon Strecker (brevet des Etats Unis d'Amérique N° 2.407.645), de la condensation selon Hofmann de l'acide chloroacétique sur des amines secondaires ou encore de l'ouverture d'époxyde convenablement choisie par une amine (M. Pascal, Compt. Rend. 1957, *244*, 1514-16).

Par ailleurs, il est connu, par le brevet des Etats Unis d'Amérique N° 3.324.123, d'accéder à des morpholinones-2 substituées à partir de diéthanolamines substituées.

En outre, dans l'arrière-plan technologique, on sait que le glyoxal opposé, d'une part, à froid, à la N-méthyl N-(hydroxy-2 éthyl) amine fournit à côté de la diméthyl-3,3'-bisoxazolidine-2,2' majoritaire, des traces de N-méthyl morpholinone-2 (5%) et de N-méthyl N-(hydroxy-2 éthyl) glycine (10%) (P. A. Laurent et al. Bull. Soc. Chim. France, *1978*, II, 83-8) et, d'autre part, un reflux en présence d'une résine KU2, la N-butyl ou N-phényl N-(hydroxy-2 éthyl) amine conduit à la dibutyl ou diphényl-4,8 octahydro oxazino-1,4[3,2-b]oxazine-1,4 (D. L. Rakhmankulov et al. brevet de l'Union Soviétique N° 565.034) hydrolysable en la glycine correspondante (A. le Rouzic et al. communication personnelle).

Ainsi, selon l'état de la technique, les procédés d'obtention des produits de formule générale (I) exigent soit la manipulation de réactifs dangereux tels que le cyanure de sodium ou l'oxyde d'éthylène, soit de longues durées réactionnelles (W. S. Gump et al., loc. cit.), soit enfin l'obtention préalable des époxydes correspondants.

Or, la demanderesse à découvert de façon tout-à-fait inattendue, un nouveau procédé général de préparation des produits de formule générale (I), obviant aux inconvénients des procédés connus, caractérisé par le fait que l'on fait réagir, à chaux, du glyoxal avec une amine hydroxylée de formule générale (III):

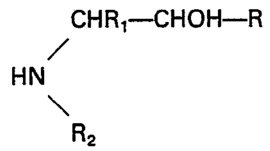

(III)

dans laquelle, R, $R_1$ et $R_2$ ont la signification donnée précédemment.

La condensation du glyoxal avec l'amine hydroxylée de formule générale (III) est exothermique et elle peut être représentée schématiquement par l'équation *1*:

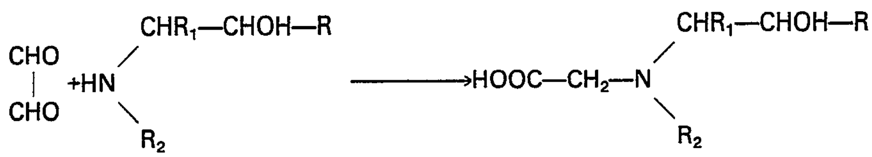

eq. 1

Intermédiairement, il peut se former la lactone du type morpholinone-2 qui s'hydrolyse aisément en glycine attendue:

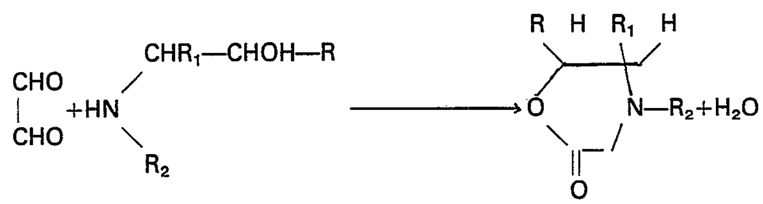

A l'état cristallisé, les produits de formule générale (I) sont préférentiellement sous forme d'un ion dipolaire (zwitterion ou amphigame).

Du point de vue des conditions opératoires, le procédé selon l'invention est relativement souple: ainsi si l'on double la proportion d'un réactif par rapport à l'autre, le rendement en glycine attendue reste toujours excellent par rapport au réactif minoritaire.

De plus, le procédé selon l'invention n'est affecté ni par une dilution aqueuse importante ni par la présence dans le milieu réactionnel d'un ou plusieurs alkanols tels que le méthanol, l'éthanol, l'éthylèneglycol. Toutefois, en milieu concentré, pauvre en eau, tel qu'il peut être obtenu, par exemple, en mettant en oeuvre du glyoxal trimère dihydraté cristallisé (tétrahydroxy-4,4',5-5' bis(dioxolanne-1,3)-2,2', selon F. Chastrette et al. Bull. Soc. Chim. France, *1983*, II, 33—40), le milieu réactionnel peut contenir, en plus ou moins grande proportion, de la lactone du type morpholinone-2 qu'il suffit de chauffer en présence d'eau pour génerer la glycine attendue. Aussi, préférentiellement, le procédé selon l'invention est effectué en milieu aqueux et avantageusement en présence de 2,5 à 7,5 moles d'eau par mole de glyoxal mis en oeuvre.

Le procédé selon l'invention est réalisé à chaud, à une température supérieure à 60°C, avantageusement à une température comprise entre 80 et 100°C et préférentiellement à 100°C.

La durée réactionnelle est fonction de la température et la vitesse réactionnelle est d'autant plus rapide que la température est plus élevée. A 100°C, la réaction est pratiquement terminée en une heure. On peut suivre l'avancement de la réaction par dosage potentiométrique de l'acide cherché sur des prises d'essai prélevées régulièrement dans le milieu réactionnel.

Le glyoxal est avantageusement mis en oeuvre sous forme de ses solutions aqueuses commerciales de 30 à 55 % en poids de glyoxal mais il est également possible d'utiliser d'autres sources de glyoxal telles que le paradioxannediol-2,3 ou le tétrahydroxy-4,4',5,5'bis(dioxolanne-1,3)-2,2'.

Les amines secondaires hydroxylées de formule générale (III) sont généralement connues et quelques unes même sont commercialisées. Habituellement, elles sont obtenues par ouverture d'un oxiranne par l'ammoniac ou une amine primaire (R. R. Wagner et H. D. Zook, Synthetic Organic Chemistry, 3ème édition, chapitre 24, John Wiley & Sons, New York, 1961). De telles amines sont, par exemple, la diéthanolamine, la bis(hydroxy-2 propyl)amine, la bis(méthyl-1 hydroxy-2 éthyl)amine, la bis (éthyl-1 hydroxy-2 éthyl)amine, la bis(diméthyl-1,2 hydroxy-2 éthyl) amine, la bis(hydroxy-2 phényl-1 éthyl)amine, la bis(hydroxy-2 phényl-2 éthyl)-amine, l'éthanolisopropanolamine, le N-méthyl aminoéthanol, l'éphédrine. Les acides

hydroxyaminocarboxyliques de formule générale (I) obtenus par le procédé de l'invention présentent de précieuses propriétés complexantes.

Les exemples suivants sont donnés à titre illustratif et nullement limitatif de l'invention.

Exemple 1

On chauffe 1 heure au reflux une solution de:

—145 g (1 mole) de glyoxal à 40 % en poids dans l'eau;

—105 g (1 mole) de diéthanolamine.

A ce stade, un dosage potentiométrique réalisé sur une prise d'essai conclut à un rendement de 96 % de la théorie.

La solution réactionnelle est alors refroidie à la température ambiante; l'acide cherché cristallise spontanément; on l'essore puis on le sèche à 60°C sous vide à poids constant.

On isole ainsi 105 g (0,65 mole) de N,N-bis(hydroxy-2 éthyl) glycine, cristallisée, présentant un point de fusion de 193° C (littérature 193°C) et un pK de 8,4 soit un rendement de 65% de la théorie.

Par concentration des eaux-méres, sous vide, aux trois quarts, on isole un deuxième jet de 36 g (0,22 mole) de N,N-bis(hydroxy-2 éthyl) glycine cristallisée présentant un point de fusion de 193°C, sans dépression en mélange avec le premier jet.

Le rendement global s'établit à 87% de la théorie.

Un échantillon recristallisé dans l'eau (F=193°C) présente la microanalyse suivant:

| | C% | H% | N% |
|---|---|---|---|
| $C_6H_{13}NO_4$ calculé | 44,16 | 8,03 | 8,58 |
| P.M. 163,16 trouvé | 43,9 | 8,5 | 8,6 |

Exemple 2

On chauffe 1 heure au reflux, une solution de:

—145 g (1 mole) de glyoxal à 40% en poids dans l'eau;

—105 g (1 mole) de diéthanolamine;

—200 $cm^3$ d'éthanol.

A ce stade, un dosage potentiométrique conclut à un rendement de 85 % de la théorie.

La solution réactionnelle est alors refroidie à la température ambiante. Le produit cherché cristallise spontanément. On l'essore et on le sèche à poids constant sous vide à 60°C. On isole ainsi un premier jet de 82 g (0.5 mole) de N,N-bis(hydroxy-2 éthyl)glycine cristallisée présentant un point de fusion de 193°C. Les eaux-mères concentrées sous vide jusqu'à moitié du volume laissent déposer un second jet de 41 g (0,25 mole) de N,N-bis(hydroxy-2 éthyl)glycine cristallisée (F=193°C).

Le rendement global s'établit à 75 % de la théorie.

Exemple 3

Dans 120 g (1 mole) de paradioxannediol-2,3 préalablement fondu puis maintenu à 80°C, on introduit sous agitation en 30 minutes: 105 g (1 mole) de diéthanolamine, puis la solution est chauffée une heure à 100°C.

A ce stade, un dosage potentiométrique conclut à un rendement de 95% de la théorie.

La solution réactionnelle est alors refroidie à la température ambiante; elle se prend en masse. On la délite sous agitation avec 175 $cm^3$ de méthanol puis on essore le produit cristallisé ainsi obtenu, en ensuite on le sèche sous vide à 60°C à poids constant.

On isole ainsi 120,7 g (0,74 mole) de N,N-bis(hydroxy-2 éthyl) glycine présentant un point de fusion de 193°C.

Le rendement global s'établit à 74% de la théorie.

Exemple 4

On chauffe 1 heure au reflux une solution de:

—210,14 g (1 mole) de tétrahydroxy-4,4',5,5' bis(dioxolanne-1,3)-2,2' cristallisé commercialisé sous le nom de glyoxal hydrate, cristallisé;

—105 g (1 mole) de diéthanolamine;

—150 $cm^3$ de méthanol,

puis le milieu réactionnel est concentré à sec sous vide.

L'huile résiduelle, analysée par spectrographie infrarouge, montre qu'elle est constituée par un mélange de N,N-bis(hydroxy-2 éthyl) glycine et de N-(hydroxy-2 éthyl)morpholone-2 caractérisées par leur absorption dans la région des carbonyles:

νc=o 1735 $cm^{-1}$ lactone

νc=o 1650 $cm^{-1}$ ion carboxylate

Cette huile est chauffée une heure au reflux dans 2 volumes d'eau.

A ce stade, un dosage potentiométrique réalisé sur une prise d'essai indique la présence de 96 % de glycine cherchée.

Le milieu réactionnel est alors refroidi à la température ambiante; l'acide cherché cristallise spontanément; on l'essore puis on le séche à 60°C sous vide à poids constant.

On isole ainsi 137 g (0,84 mole) de N,N-bis(hydroxy-2 éthyl) glycine présentant un point de fusion de 193°C.

Exemple 5

On chauffe une heure au reflux une solution de:

—145 g (1 mole) de glyoxal à 40% en poids dans l'eau;

—210 g (2 moles) de diéthanolamine.

A ce stade, un dosage potentiométrique réalisé sur une prise d'essai conclut à un rendement de 90 % de la théorie.

Puis la solution réactionnelle est traitée comme dans l'exemple 1. On isole ainsi un premier jet de 73,4 g (0,45 mole) de N,N-bis (hydroxy-2 éthyl)glycine cristallisée présentant un point de fusion de 193°C. On n'a pas cherché à isoler un deuxième jet.

Exemple 6

On chauffe une heure au reflux une solution de:

—290 g (2 moles) de glyoxal à 40 % en poids dans l'eau;

—105 g (1 mole) de diéthanolamine.

A ce stade, un dosage potentiométrique réalisé sur une prise d'essai conclut à un rendement de 95 % de la théorie.

Puis, la solution réactionnelle est traitée comme dans l'exemple 1. On isole ainsi un premier jet de 90 g (0,55 mole) de N,N-bis(hydroxy-2 éthyl)glycine cristallisée présentant un point de fusion de 193°C. On n'a pas cherché à isoler un deuxième jet.

Exemple 7

On chauffe 1 heure au reflux une solution de:

—145 g (1 mole) de glyoxal à 40 % en poids dans l'eau,

—133,2 g (1 mole) de bis(hydroxy-2 propyl)amine.

A ce stade, un dosage potentiométrique réalisé sur une prise d'essai conclut à un rendement de 93 % de la théorie.

La solution réactionnelle est ensuite traitée comme dans l'exemple 1. On isole ainsi en deux jets, 155 g (0,81 mole) de N,N-bis (hydroxy-2 propyl)glycine présentant un point de fusion de 145°C et un pKa de 8,2 (littérature M. L. Pascal, Bull. Soc. Chim. France, 1960, II, 435-42, F=145—146°C).

Le rendement global s'établit à 81 % de la théorie.

Exemple 8

On chauffe 1 heure au reflux une solution de:

—29 g (0,2 mole) de glyoxal à 40 % en poids dans l'eau,

—33 g (0,2 mole) d'éphédrine racémique,

—150 $cm^3$ d'éthanol,

—200 $cm^3$ d'eau,

puis on distille l'éthanol sous vide et l'on poursuit ensuite le chauffage au reflux pendant une heure.

A ce stade, un dosage potentiométrique effectué sur une prise d'essai révèle la présence de 100 % d'acide cherché.

La solution aqueuse est ensuite concentrée à sec sous vide; l'huile résiduelle est empâtée au reflux dans 250 $cm^3$ d'isopropanol puis elle est abandonnée 3 heures à 0°C. Le précipité cristallisé est ensuite essoré puis séché sous vide à 60°C à poids constant.

On isole ainsi 30 g (0,135 mole) de N-méthyl N-(hydroxy-2 méthyl-1 phényl-2 éthyl)glycine cristallisée présentant un point de fusion 163°C.

Le rendement global s'établit à 67% de la théorie.

Microanalyse

| | C% | H% | N% |
|---|---|---|---|
| $C_{12}H_{17}NO_3$ calculé | 64,55 | 7,68 | 6,27 |
| P.M. 223,3 trouvé | 64,5 | 7,8 | 6,1 |

A la connaissance de la demanderesse, ce produit n'est pas décrit dans la littérature.

Exemple 9

On chauffe 1 heure au reflux une solution de:

—145 g (1 mole) de glyoxal en solution aqueuse à 40% en poids;

—75,1 g (1 mole) de méthyléthanolamine;

—100 g d'eau.

A ce stade, un dosage potentiométrique réalisé sur une prise d'essai indique la présence de 99 % d'acide cherché.

La solution est ensuite concentrée sous vide de manière à éliminer 82 g d'eau puis le milieu réactionnel est abandonné à la cristallisation pendant 16 heures à la température ambiante.

Ensuite, la masse cristallisée obtenue est délitée avec 250 $cm^3$ d'éthanol puis on l'essore et le produit recueillie est ensuite séché à 60°C sous vide à poids constant.

On isole ainsi 99 g (0,74 mole) de N-méthyl N(hydroxy-2 éthyl) glycine cristallisée présentant un point de fusion de 132°C. (littérature E. Knorr et al. Annalen, 1899, *307*, 201, F=132—133°C).

Exemple 10

On chauffe 1 heure au reflux une solution de:

—14,5 g (0,1 mole) de glyoxal à 40 % en poids dans l'eau;

—25,7 g (0,1 mole) de tétradécylamino-2 éthanol dans 100 $cm^3$ d'éthanol.

A ce stade, un échantillon analysé par spectrographie infrarouge montre la présence d'une fonction lactone. On introduit alors dans la solution réactionnelle 160 g d'eau, puis on poursuit le reflux pendant 2 heures.

La solution réactionnelle est ensuite concentrée sous vide, l'acide cherché cristallise. On l'isole puis on le recristallise par chaud et froid dans du dioxanne. On obtient ainsi 24,1 g de N-(hydroxy-2 éthyl) N-tétradécyl glycine cristallisée, présentant un point de fusion de 95°C, soit un rendement de 76,5 % de la théorie.

Microanalyse

| | C% | H% | N% | O% |
|---|---|---|---|---|
| $C_{18}H_{37}NO_3$ Calculés | 68,52 | 11,82 | 4,44 | 15,21 |
| PM=315,5 Trouvés | 68,6 | 12,4 | 4,2 | |

Il va de soi que la présente invention n'a été décrite qu'à titre purement explicatif et nullement limitatif et que toute modification utile pourra y être apportée sans sortir de son cadre.

**Revendications**

1. Procédé de préparation d'acides hydroxyalkylaminoacétiques de formule générale (I):

$$R—CHOH—CHR_1—NR_2—CH_2—COOH \qquad (I)$$

dans laquelle:

R représente un atome d'hydrogène, un groupement alkyle en $C_1$—$C_{18}$, à chaîne droite ou ramifiée, ou un groupement phényle éventuellement substitué par un ou plusieurs radicaux (de préférence un ou deux) choisis parmi les radicaux: alkyle en $C_1$—$C_4$, alcoxyle en $C_1$—$C_4$ ou halogéno;

$R_1$ représente un atome d'hydrogène ou un groupement alkyle en $C_1$—$C_4$;

$R_2$ représente un groupement alkyle en $C_1$—$C_{18}$ ou le groupement $R_3$—CHOH—$CHR_4$— dans lequel $R_3$, identique à R, représente un atome d'hydrogène ou un groupement alkyle en $C_1$—$C_{18}$, à chaîne droite ou ramifiée et $R_4$ est identique à $R_1$ caractérisé par le fait que l'on fait réagir, à chaud, à une température supérieure à 60°C, du glyoxal sur une amine secondaire hydroxylée de formule générale (III):

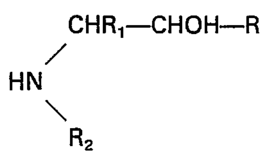

(III)

dans laquelle R, $R_1$ et $R_2$ ont la signification donnée ci-dessus.

2. Procédé selon la revendication 1, caractérisé par le fait qu'il est effectué en présence d'eau.

3. Procédé selon la revendication 1 ou 2, caractérisé par le fait que l'on fait réagir à une température comprise entre 60 et 100°C, une mole de glyoxal en solution aqueuse sur une mole d'amine secondaire hydroxylée de formule générale (III) ci-dessus.

4. Procédé selon l'une quelconque des revendications 1 à 3, caractérisé par le fait que l'acide hydroxyalkylaminoacétique de formule générale (I) est un acide de formule générale (II):

$R_3$—CHOH—$CHR_4$
\
N—$CH_2$—COOH (II)
/
$R_3$—CHOH—$CHR_4$

dans laquelle $R_3$ représente un atome d'hydrogène ou un groupement alkyle à chaîne droite ou ramifiée en $C_1$—$C_{18}$ et $R_4$ représente un atome d'hydrogène ou un groupement alkyle en $C_1$—$C_4$.

5. Procédé selon l'une quelconque des revendications 1 à 3, caractérisé par le fait que l'acide hydroxyalkylaminoacétique est la N-méthyl N-(hydroxy-2 éthyl)glycine.

6. Procédé selon l'une quelconque des revendications 1 à 3, caractérisé par le fait que l'acide hydroxyalkylaminoacétique est la N-méthyl N-(hydroxy-2 méthyl-1 phényl-2 éthyl) glycine.

7. Procédé selon la revendication 4, caractérisé par le fait que l'acide hydroxyalkylaminoacétique est la N,N-bis(hydroxy-2 éthyl)glycine.

8. Procédé selon la revendication 1, caractérisé par le fait que l'acide hydroxyalkylaminoacétique est la N,N-bis(hydroxy-2 propyl)glycine.

**Patentansprüche**

1. Verfahren zur Herstellung von Hydroxyalkylaminoessigsäuren der allgemeinen Formel (I):

R—CHOC—$CHR_1$—$NR_2$—$CH_2$—COOH (I)

worin:

R ein Wasserstoffatom, eine geradkettige oder verzweigte $C_1$—$C_{18}$-Alkylgruppe, oder eine Phenylgruppe, welche gegebenenfalls mit einem oder mehreren (vorzugsweise einem oder zwei) Resten, ausgewählt unter $C_1$—$C_4$-Alkylresten, $C_1$—$C_4$-Alkoxylresten oder Halogenresten, substituiert sein kann, darstellt;

$R_1$ ein Wasserstoffatom oder eine $C_1$—$C_4$-Alkylgruppe darstellt;

$R_2$ eine $C_1$—$C_{18}$-Alkylgruppe oder die Gruppe $R_3$—CHOH—$CHR_4$—, worin $R_3$, identisch mit R, ein Wasserstoffatom oder eine geradkettige oder verzweigte $C_1$—$C_{18}$-Alkylgruppe darstellt und $R_4$ mit $R_1$ identisch ist, bedeutet; dadurch gekennzeichnet, daß man in der Wärme bei einer Temperatur oberhalb von 60° C Glyoxal auf ein sekundäres, hydroxyliertes Amin der allgemeinen Formel (III):

$CHR_1$—CHOH—R
/
HN (III)
\
$R_2$

worin R, $R_1$ und $R_2$ die zuvor angegebenen Bedeutungen haben, einwirken läßt.

2. Verfahren nach Anspruch 1, dadurch gekennzeichnet, daß es in Anwesenheit von Wasser durchgeführt wird.

3. Verfahren nach Anspruch 1 oder 2, dadurch gekennzeichnet, daß man bei einer Temperatur zwischen 60 und 100°C 1 Mol Glyoxal in wässriger Lösung mit 1 Mol sekundärem, hydroxyliertem Amin der vorgenannten allgemeinen Formel (III) reagieren läßt.

4. Verfahren nach einem der Ansprüche 1 bis 3, dadurch gekennzeichnet, daß die Hydroxyalkylaminoessigsäure der allgemeinen Formel (I) eine Säure der allgemeinen Formel (II) ist:

$R_3$—CHOC—$CHR_4$
\
N—$CH_2$—COOH (II)
/
$R_3$—CHOH—$CHR_4$

worin $R_3$ ein Wasserstoffatom oder eine geradkettige oder verzweigte $C_1$—$C_{18}$-Alkylgruppe bedeutet und $R_4$ ein Wasserstoffatom oder ein $C_1$—$C_4$-Alkylgruppe bedeutet.

5. Verfahren nach einem der Ansprüche 1 bis 3, dadurch gekennzeichnet, daß die Hydroxyalkylaminoessigsäure N-Methyl-N-(2-hydroxyethyl)-glycin ist.

6. Verfahren nach einem der Ansprüche 1 bis 3, dadurch gekennzeichnet, daß die Hydroxyalkylaminoessigsäure N-Methyl-N-(2-hydroxy-1-methyl-2-phenylethyl)-glycin ist.

7. Verfahren nach Anspruch 4, dadurch gekennzeichnet, daß die Hydroxyalkylamino-essigsäure N,N-bis-(2-Hydroxyethyl)-glycin ist.

8. Verfahren nach Anspruch 1, dadurch gekennzeichnet, daß die Hydroxyalkylamino-essigsäure N,N-bis-(2-Hydroxy-propyl)-glycin ist.

**Claims**

1. Process for the preparation of hydroxyalkylamino-acetic acids of general formula (I):

$$R{-}CHOH{-}CHR_1{-}NR_2{-}CH_2{-}COOH \qquad (I)$$

in which:

R represents a hydrogen atom, a straight-chain or branched $C_1$—$C_{18}$ alkyl group, or a phenyl group substituted, if required, with one or more radicals (preferably, one or two radicals) chosen from amongst the radicals: $C_1$—$C_4$ alkyl, $C_1$—$C_4$ alkoxy or halo;

$R_1$ represents a hydrogen atom or a $C_1$—$C_4$ alkyl group;

$R_2$ represents a $C_1$—$C_{18}$ alkyl group or the group $R_3$—CHOH—$CHR_4$— in which $R_3$, which is identical to R, represents a hydrogen atom or a straight-chain or branched $C_1$—$C_{18}$ alkyl group and $R_4$ is identical to $R_1$, characterized in that glyoxal is reacted, in the heated state, at a temperature greater than 60°C, with a hydroxylated secondary amine of general formula (III):

$$HN\begin{matrix} \diagup CHR_1{-}CHOH{-}R \\ \diagdown R_2 \end{matrix} \qquad (III)$$

in which R, $R_1$ and $R_2$ have the meaning given above.

2. Process according to Claim 1, characterized in that it is carried out in the presence of water.

3. Process according to Claim 1 or 2, characterized in that one mole of glyoxal dissolved in water is reacted with one mole of the hydroxylated secondary amine of general formula (III) above, at a temperature between 60 and 100°C.

4. Process according to any one of Claims 1 to 3, characterized in that the hydroxyalkylaminoacetic acid of general formula (I) is an acid of general formula (II):

$$\begin{matrix} R_3{-}CHOH{-}CHR_4 \diagdown \\ R_3{-}CHOH{-}CHR_4 \diagup \end{matrix} N{-}CH_2{-}COOH \qquad (II)$$

in which $R_3$ represents a hydrogen atom or a straight-chain or branched $C_1$—$C_{18}$ alkyl group and $R_4$ represents a hydrogen atom or a $C_1$—$C_4$ alkyl group.

5. Process according to any one of Claims 1 to 3, characterized in that the hydroxyalkylaminoacetic acid is N-methyl-N-(2-hydroxyethyl)glycine.

6. Process according to any one of Claims 1 to 3, characterized in that the hydroxyalkylaminoacetic acid is N-methyl-N-(2-hydroxy-1-methyl-2-phenylethyl)glycine.

7. Process according to Claim 4, characterized in that the hdroxyalkylaminoacetic acid is N,N-bis(2-hydroxyethyl)glycine.

8. Process according to Claim 1, characterized in that the hydroxyalkylaminoacetic acid is N,N-bis(2-hydroxypropyl)glycine.